# EUROPÄISCHE PATENTANMELDUNG

(11) **EP 2 952 216 A1**
(43) Veröffentlichungstag der Anmeldung: **09.12.2015**
(21) Anmeldenummer: 15170510.0
(22) Anmeldetag: 03.06.2015
(51) Int. Cl.: A61M 5/14, A61J 3/00, B01F 3/08, G05D 11/13

(54) **VORRICHTUNG ZUR BEREITSTELLUNG EINER AUS MEHREREN KOMPONENTEN BESTEHENDEN MISCHLÖSUNG**

(30) Priorität: 05.06.2014 DE 102014210772; 05.06.2014 DE 202014102632 U; 09.03.2015 DE 202015101150 U
(71) Anmelder: Hemedis GmbH, 09638 Lichtenberg (DE)
(72) Erfinder: Hegewald, Robert, 09638 Lichtenberg (DE)
(74) Vertreter: Kailuweit & Uhlemann Patentanwälte Partnerschaft mbB

(57) **Zusammenfassung**

Die Erfindung betrifft Vorrichtungen zur Bereitstellung einer aus mehreren Komponenten bestehenden Mischlösung. Die Mischlösungen sind dabei einfach bereitstellbar.

Dazu weist die Vorrichtung
- Behälter mit flüssigen Komponenten und damit über erste Leitungen verbundene Fördereinrichtungen für die Komponenten,
- einen Sammelbehälter für die Mischlösung über zweite Leitungen in Verbindung mit den Fördereinrichtungen,
- jeweils mindestens vier Ultraschallsensoren als Sender-Detektor-Kombinationen für eine Komponente einer Leitung kreuzweise durchdringende Schallwellen und
- ein mit den Fördereinrichtungen und den Ultraschallsensoren verbundenes Datenverarbeitungssystem auf.

Die Ultraschallsensoren sind sowohl Sender als auch Empfänger. Die Zeiten und die Intensitäten der Schallwellen sind damit leicht miteinander und der jeweiligen Komponente zuordenbar. Das Datenverarbeitungssystem ist ein aus den Zeiten und den Signalen der Ultraschallsensoren den jeweiligen Fluss der Komponente in der Leitung ermittelndes, mit vorbestimmten Mengen der Komponenten vergleichendes und im Ergebnis die jeweilige Fördereinrichtung entsprechend der vorbestimmten Menge der Komponente an der Mischlösung steuerndes Datenverarbeitungssystem.

## Beschreibung

Die Erfindung betrifft Vorrichtungen zur Bereitstellung einer aus mehreren Komponenten bestehenden Mischlösung.

Einrichtungen zur Bereitstellung von Mischlösungen sind unter anderem aus der Medizintechnik als Compounder bekannt. Zur dosierten Zuführung einzelner Komponenten werden auch bekannte Spritzenpumpen verwendet. Diese bestehen im Wesentlichen aus einem zylindrischen Hohlraum mit einem beweglichen Kolben. Der Zylinder weist eine konusförmige Düse für den Ein- und Austritt einer Flüssigkeit auf. Bei der Spritzenpumpe ist der Kolben an einen translatorisch wirkenden Antrieb gekoppelt. Mit der Bewegung des Kolbens kann Flüssigkeit eingesaugt oder ausgepresst werden.

Durch die Druckschrift US 2002/0064880 A1 ist ein Fluidabgabesystem mit einer Vorrichtung zum Steuern der Strömung von Fluiden durch eine Mehrzahl von Ventilen bekannt. Die Strömung kann durch einen Sensor erfasst werden. Die Menge der einzelnen Fluide wird über das Ventil bestimmt, wobei deren Betätigungseinrichtungen an einen Nocken gekoppelt sind.

Die Druckschrift EP 0 543 259 A1 beinhaltet ein Mehrfachinfusionssystem zur dosierten, kontinuierlichen, gleichzeitigen Infusion von mehreren Infusions- oder Medikamentenlösungen. Ein Bestandteil ist dabei eine Schwerkraftinfusionseinrichtung, die mit einem Tropfdetektor versehen ist, der nicht weiter ausgeführt ist. Weiterhin sind Druckinfusionsapparate vorgesehen, die Alarmsignale ausgeben können.

Durch die Druckschrift EP 0 343 501 A2 ist ein Mehrfachinfusionssystem bekannt, wobei ein Durchflusselement vorgesehen ist, welches bei Erkennen eines Flussstopps die Steuereinrichtung veranlasst, die gesamte Vorrichtung zu deaktivieren. Die Vorrichtung umfasst dabei Behälter, die über jeweils eine Pumpe mit dem Durchflusselement verbunden sind. Die Förderrate wird durch eine Rechen- und Steuereinheit entsprechend einem vorbestimmten Programm geregelt, welches nicht weiter ausgeführt ist.

Der im Patentanspruch 1 angegebenen Erfindung liegt die Aufgabe zugrunde, eine aus mehreren Komponenten bestehende Mischlösung einfach bereitzustellen.

Diese Aufgabe wird mit den im Patentanspruch 1 aufgeführten Merkmalen gelöst.

Die Vorrichtungen zur Bereitstellung einer aus mehreren Komponenten bestehenden Mischlösung zeichnen sich insbesondere dadurch aus, dass Mischlösungen einfach bereitstellbar sind.

Dazu weist die Vorrichtung
- Behälter mit flüssigen Komponenten und damit über erste Leitungen verbundene Fördereinrichtungen für die Komponenten,
- einen Sammelbehälter für die Mischlösung über zweite Leitungen in Verbindung mit den Fördereinrichtungen,
- jeweils mindestens vier Ultraschallsensoren als Sender-Detektor-Kombinationen für eine Komponente einer Leitung kreuzweise durchdringende Schallwellen und
- ein mit den Fördereinrichtungen und den Ultraschallsensoren verbundenen Datenverarbeitungssystem auf.

Die eingesetzten Ultraschallsensoren sind sowohl Sender als auch Empfänger. Die Zeiten und die Intensitäten der Schallwellen sind damit leicht miteinander und der jeweiligen Komponente zuordenbar. Die Intensitäten können dazu sowohl die des Senders als auch die des Empfängers sein. Darüber hinaus ist das Datenverarbeitungssystem ein aus den Zeiten und den Signalen der Ultraschallsensoren jeweils als Sender und Empfänger den jeweiligen Fluss der Komponente in der Leitung ermittelndes, mit vorbestimmten Mengen der Komponenten vergleichendes und im Ergebnis die jeweilige Fördereinrichtung entsprechend der vorbestimmten Menge der Komponente an der Mischlösung steuerndes Datenverarbeitungssystem.

Damit steht dem Nutzer eine einfach zu bedienende und universell einsetzbare Vorrichtung zur Bereitstellung einer aus mehreren Komponenten bestehenden Mischlösung zur Verfügung. Universell bezieht sich auf der Möglichkeit der leichten Kombinierbarkeit. Die Vorrichtung kann durch ihre Realisierung leicht entsprechend der Anzahl der Komponenten für die Mischlösung realisiert werden. Derartige Mischlösungen können insbesondere Mischlösungen zur parenteralen Ernährung oder Infusionslösungen sein.

Vorteilhafterweise werden Ultraschallsensoren zur Messung des Flusses der Komponenten an jeder der die Behälter mit den Fördereinrichtungen verbindenden Leitungen verwendet. Als Leitungen können dazu vorteilhafterweise Schlauchleitungen, Rohrstücke oder Kombinationen daraus eingesetzt werden. Die Detektion von fließenden Komponenten vereinfacht sich wesentlich, da die Ultraschallsensoren eingehaust realisiert sind. Fehler aus Verschmutzungen, die bei optischen Systemen zu Fehlmessungen führen können, werden vermieden. Besondere Ausgestaltungen oder Anforderungen an die Leitungen der Komponenten sind nicht notwendig. Damit können bekannte Schlauchleitungen und/oder Rohrstücke insbesondere aus einem Kunststoff als Leitungen für die Komponenten eingesetzt werden. Über den Fluss der Komponenten kann leicht vorbestimmte Mengen und/oder Volumina der Komponenten für die zu realisierende Mischlösung kontrolliert realisiert werden. Damit sind die Komponenten entsprechend ihrer Anteile an der Mischlösung über deren Fluss in der jeweiligen Leitung dem Behälter für die Mischlösung zuführbar. Grundlage sind die aus den Behältern fließenden und zu fördernden Komponenten. Damit können die Mischlösungen leicht genau dosiert werden. Bei Erreichen der Menge aus dem Volumen wird die Förderung der jeweiligen Komponente gestoppt. Die Bestimmung des Flusses der Komponenten gewährleistet eine sichere Herstellung der Mischlösung. Fehler, insbesondere durch das Nichtvorhandensein wenigstens einer der Komponenten hervorgerufen, werden vermieden. Bei Fehlen von Komponenten wird der Herstellungsprozess der Mischlösung gestoppt. In Ergänzung können auch vorteilhafterweise Rückschlagventile eingesetzt werden, für die keine Ansteuerung erforderlich sind. Das können auch Dreiwegeventile sein, die auch leicht durch zwei miteinander verbundene Rückschlagventile realisiert werden können.

Vorteilhafte Ausgestaltungen der Erfindung sind in den Patentansprüchen 2 bis 15 angegeben.

Das Datenverarbeitungssystem ist nach der Weiterbildung des Patentanspruchs 2 ein aus den Zeiten und den Signalen der Ultraschallsensoren die jeweilige Viskosität der in der Leitung fließenden Komponente ermittelndes Datenverarbeitungssystem. Damit ergibt sich eine weitere Möglichkeit der Überwachung bei der Realisierung der Mischlösung. Eine mit einer Veränderung der Viskosität einhergehende Veränderung einer Komponente kann leicht während der Zuführung der Komponente ermittelt werden. Die Mischlösung ist in dieser Hinsicht leicht kontrollierbar.

Das Datenverarbeitungssystem ist nach der Weiterbildung des Patentanspruchs 3 ein die Differenzen und/oder die Mittelwerte aus den Zeiten und den Signalen der Ultraschallsensoren ermittelndes Datenverarbeitungssystem, wobei daraus die jeweilige Viskosität der in der Leitung fließenden Komponente und/oder der jeweilige in der Leitung fließende Stoff bestimm- und zuordenbar ist. Insbesondere kann damit der der Mischlösung zugeführte Stoff als Komponente vorteilhafterweise kontrolliert werden.

Der Behälter und/oder eine mit dem Behälter verbundene Tropfkammer weist nach der Weiterbildung des Patentanspruchs 4 jeweils einen das Vorhandensein der Komponente detektierender Sensor auf. Weiterhin ist der das Vorhandensein der Komponente detektierende Sensor mit dem Datenverarbeitungssystem verbunden, wobei das Datenverarbeitungssystem ein wenigstens das Nichtvorhandensein der Komponente signalisierendes und die zugeordnete Fördereinrichtung stoppendes Datenverarbeitungssystem ist.

Der das Vorhandensein der Komponente detektierende Sensor ist nach der Weiterbildung des Patentanspruchs 5 vorteilhafterweise ein Ultraschallsensor, wobei die Leitung, die Tropfkammer und/oder der Behälter zwischen dem Ultraschallsender und dem Ultraschallempfänger für ein Durchschallungsverfahren oder die Leitung, die Tropfkammer und/oder der Behälter zwischen entweder den nebeneinander angeordneten Ultraschallsender und Ultraschallempfänger oder einer Kombination aus Ultraschallsender und Ultraschallempfänger und einem Reflektor für Ultraschallwellen für ein Reflexionsschallverfahren angeordnet sind. Der Fluss der Komponente in den Leitungen und das Vorhandensein der Komponente in den Behältern und/oder in den Tropfkammern ist damit einfach und sicher detektierbar. Gleichzeitig kann vorteilhafterweise das Auftreten von Luftblasen erfasst werden, so dass bei deren Auftreten das Herstellen der Mischlösung gestoppt werden kann. Damit sind auch Mischlösungen ohne Luftblasen einfach und sicher bereitstellbar.

Die Ultraschallsensoren für eine Komponente befinden sich nach der Weiterbildung des Patentanspruchs 6 jeweils in einem ring- oder u-förmigen Halteelement. Diese sind getrennt voneinander fixierbar oder die Halteelemente sind zusammen ein Halter, so dass auch eine kompakte Realisierung gegeben ist.

Die Fördereinrichtungen sind nach der Weiterbildung des Patentanspruchs 7 angetriebene Spritzenpumpen, die jeweils über ein Ventil mit dem Behälter für die Mischlösung verbunden sind. Mittels Spritzenpumpen sind leicht auch kleinste Mengen förderbar.

Der Rotor eines Schrittmotors als Antrieb der Spritzenpumpe ist nach der Weiterbildung des Patentanspruchs 8 über eine Gewindespindel und eine Bewegungsmutter auf der Gewindespindel mit dem Kolben der Spritze der Spritzenpumpe gekoppelt, wobei die Gewindespindel ein Bestandteil eines Gestells ist. Eine Einrichtung zur Abstandsmessung ist weiterhin zwischen wenigstens einem Teil des Gestells und der Bewegungsmutter zur Bestimmung der Position der Bewegungsmutter und der daraus resultierenden Position des Kolbens der Spritze der Spritzenpumpe angeordnet. Diese Einrichtung ist darüber hinaus mit dem Datenverarbeitungssystem zusammengeschaltet, so dass das Datenverarbeitungssystem ein das durch die Spritzenpumpe geförderte Volumen der Komponente bestimmendes Datenverarbeitungssystem ist.

Jeweils auf wenigstens einem Bereich des zylindrischen Hohlraums der Spritze der Spritzenpumpe für die Komponente ist nach der Weiterbildung des Patentanspruchs 9 ein das Vorhandensein der Komponente detektierender Ultraschallsensor angeordnet. Die Ultraschallsensoren der Spritzenpumpen sind weiterhin mit dem Datenverarbeitungssystem zusammengeschaltet. Durch ein Nichtvorhandensein einer zu fördernden Komponente hervorgerufene Fehldosierungen in der Mischlösung werden vermieden.

Das Datenverarbeitungssystem ist nach der Weiterbildung des Patentanspruchs 10 mit wenigstens einer Dateneingabeeinrichtung, einem Bildschirm und mindestens einer Schnittstelle verbunden.

Die Vorrichtung umfasst nach der Weiterbildung des Patentanspruchs 11
- wenigstens ein erstes Modul mit Behältern mit flüssigen Komponenten,
- mindestens ein zweites Modul mit angetriebenen Spritzenpumpen und mit elektrischen Verbindungselementen für Energie- und Datenleitungen für ein vor- und ein nachordenbares weiteres zweites Modul, wobei die Spritzenpumpe einen ersten mit dem Antrieb gekoppelten Halter in Verbindung mit dem Kolben und einen zweiten Halter für entweder den Zylinder der Spritzenpumpe oder einen Adapter in Verbindung mit dem Zylinder der Spritzenpumpe aufweist und der Adapter eine Vertiefung zur lösbaren Fixierung für ein Leitungsstück und eine Aufnahme für einen Bereich einer Schlauchleitung besitzt,
- ein drittes Modul als Waage für den die Mischlösung aufnehmenden Behälter und
- ein viertes Modul mit einer Steuereinrichtung als Datenverarbeitungssystem, wobei zum Einen jeweils ein Behälter (5) über wenigstens ein Ventil mit einer Spritzenpumpe und die jeweilige Spritzenpumpe über das Ventil oder ein weiteres Ventil mit dem die Mischlösung aufnehmenden Behälter als Sammelbehälter über Schlauchleitungen verbunden sind und zum Anderen die Antriebe der Spritzenpumpen und die Waage mit der Steuereinrichtung zusammengeschaltet sind.

Die Einrichtung zeichnet sich durch ihre einfache Realisierung und ihre Kombinierbarkeit aus. Entsprechend der zu realisierenden Mischlösung können die dazu notwendigen Module mit den Komponenten einfach bereitgestellt werden. Die Einrichtung kann durch den modularen Aufbau leicht entsprechend der Anzahl der Komponenten für die Mischlösung realisiert werden. Derartige Mischlösungen können insbesondere Mischlösungen zur parenteralen Ernährung oder Infusionslösungen sein.

Der Transport der Komponenten zum Behälter für die Mischlösung erfolgt mittels der Spritzenpumpen. Vorteilhafterweise können Spritzen verschiedener Größe angebracht werden, so dass auch kleine Mengen einer Komponente dem Behälter für die Mischlösung mengengenau mittels in ihren Abmessungen kleinen Spritzen zugeführt werden können. Ansonsten bei großen Spritzen notwendige kleinste Stellwege für den Kolben der Spritze werden vermieden. Dazu ist der Adapter vorgesehen, so dass keine Umbauarbeiten notwendig sind. Damit können am zweiten Modul in ihren Abmessungen größere und kleinere Spritzen einfach angeordnet werden.

Die Zuführung der Komponenten zum Behälter der Mischlösung erfolgt mittels der Spritzenpumpen, wobei über den Weg des Kolbens und dem Querschnitt des Zylinders der Spritze ein bestimmtes Volumina dem Behälter der Mischlösung zugeführt werden kann. Vorteilhafterweise befindet sich der Behälter für die Mischlösung auf der Waage des dritten Moduls, so dass mittels der Steuereinrichtung des vierten Moduls eine weitere Kontrolle der Mischlösung gegeben ist. Damit kann mit der Einrichtung eine Mischlösung aus verschiedenen Komponenten sicher bereitgestellt werden.

Das Ventil kann ein bekanntes Dreiwegeventil sein. In zwei der drei Wege können auch einfache Ventile als Rückschlagventile angeordnet sein, so dass eine Verbindung zwischen Behälter mit flüssiger Komponente und Spritze oder Spritze zum Behälter für die Mischlösung vorhanden ist.

Der Adapter ist nach der Weiterbildung des Patentanspruchs 12 ein Zylinder in Verlängerung der Spritze der Spritzenpumpe in Längsrichtung. Der Zylinder weist eine Längsnut für einen Verbinder oder ein Dreiwegeventil als Ventil und als Aufnahme für den Bereich der Schlauchleitung auf. Die Aufnahme besitzt eine Schräge, so dass die Schlauchleitung nach außen geführt ist. Der Zylinder weist nach der Schräge eine Befestigungseinrichtung für den zweiten Halter auf. Die Vertiefung zur lösbaren Fixierung des Leitungsstückes des Verbinders oder Dreiwegeventils und die Längsnut sind ein Hohlraum, wobei die Vertiefung und Längsnut winklig zueinander angeordnet sind.

Nach der Weiterbildung des Schutzanspruchs 13 sind entweder die vom Zylinder nach außen geführte Schlauchleitung mit einem Behälter oder einer Tropfkammer in Verbindung mit einem Behälter und die mit dem Leitungsstück in der Vertiefung verbundene Schlauchleitung mit dem die Mischlösung aufnehmenden Behälter oder die vom Zylinder nach außen geführte Schlauchleitung mit dem die Mischlösung aufnehmenden Behälter und die mit dem Leitungsstück in der Vertiefung verbundene Schlauchleitung mit einem Behälter oder einer Tropfkammer in Verbindung mit einem Behälter verbunden.

Der Verbinder ist nach der Weiterbildung des Schutzanspruchs 14 ein T-Verbinder mit drei Anschlüssen. Das Leitungsstück in der Vertiefung ist der erste Anschluss, der zweite Anschluss ist mit der Spritzenpumpe verbunden und der dritte Anschluss befindet sich in der Längsnut. Der T-Verbinder kann dazu vorteilhafterweise ein einstückiges Kunststoffteil sein. Das Leitungsstück sichert dabei die Fixierung im Adapter insbesondere auch bei der Bewegung des Kolbens der Spritze. Das Leitungsstück ist dazu in der Vertiefung geklemmt.

Der erste Halter weist nach der Weiterbildung des Schutzanspruchs 15 ein Reduzierstück für die Öffnung auf. Dieses kann ebenfalls eine U-Form aufweisen, so dass die Vertiefung des Halters in ihrem Querschnitt verkleinert ist. Dadurch ist auch damit ein Wechsel der Spritze einfach möglich.

Ein Ausführungsbeispiel der Erfindung ist in den Zeichnungen jeweils prinzipiell dargestellt und wird im Folgenden näher beschrieben.

Es zeigen:
- Fig. 1: eine Vorrichtung zur Bereitstellung einer aus mehreren Komponenten bestehenden Mischlösung,
- Fig. 2: eine Haltevorrichtung für und damit mit ersten Ultraschallsensoren für den Fluss einer Komponente und zweiten Ultraschallsensoren für das Vorhandensein einer Komponente,
- Fig. 3: eine Spritzenpumpe mit einem dritten Ultraschallsensor,
- Fig. 4: eine Einrichtung für mehrere Spritzenpumpen,
- Fig. 5: ein Adapter für eine Spritzenpumpe,
- Fig. 6: den Adapter in einer Schnittdarstellung und
- Fig. 7: einen Adapter in Verbindung mit einer Spritzenpumpe.

Eine Vorrichtung zur Bereitstellung einer aus mehreren Komponenten bestehenden Mischlösung besteht im Wesentlichen aus Behältern 1 mit flüssigen Komponenten, Fördereinrichtungen 2 für die Komponenten, einem Sammelbehälter 3 für die Mischlösung, Ultraschallsensoren und einem Datenverarbeitungssystem 4.

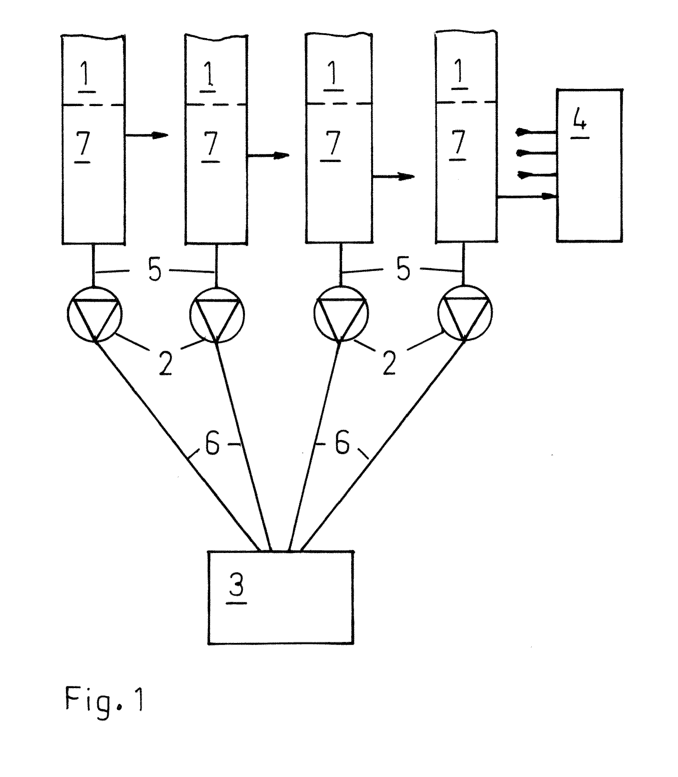

Die Fig. 1 zeigt eine Vorrichtung zur Bereitstellung einer aus mehreren Komponenten bestehenden Mischlösung in einer prinzipiellen Darstellung.

Die Behälter 1 sind über erste Leitungen 5 mit den Fördereinrichtungen 2 und diese über zweite Leitungen 6 mit dem Sammelbehälter 3 verbunden.

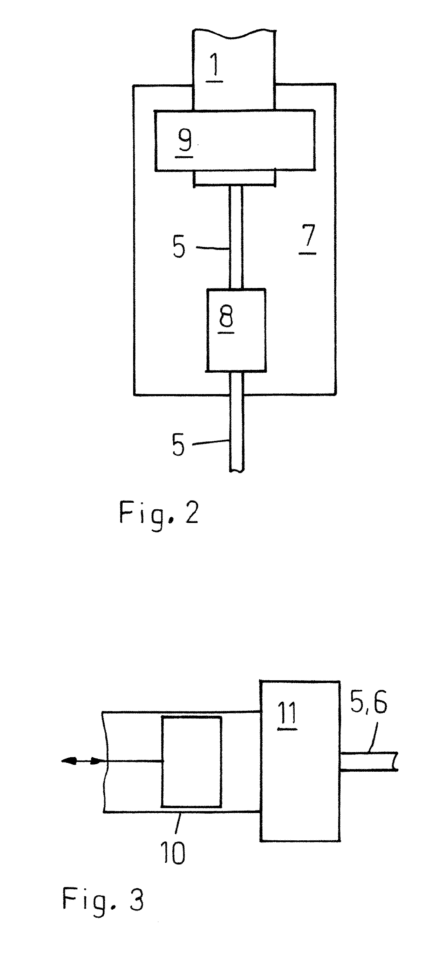

Die Fig. 2 zeigt eine Haltevorrichtung 7 für und damit mit ersten Ultraschallsensoren 8 für den Fluss einer Komponente und zweiten Ultraschallsensoren 9 für das Vorhandensein einer Komponente in einer prinzipiellen Darstellung.

Mindestens vier Ultraschallsensoren 8 sind als Sender-Detektor-Kombinationen für eine Komponente erste Bestandteile einer Haltevorrichtung 7 zur Erfassung des Flusses der Komponente in der ersten Leitung 5. Diese wird damit kreuzweise von Schallwellen durchdrungen. Diese Ultraschallsensoren 8 sind sowohl Sender als auch Empfänger und damit Detektoren der Schallwellen. Die vier Ultraschallsensoren 8 sind vier erste Ultraschallsensoren 8 einer Vorrichtung. Der Behälter 1 und/oder eine mit dem Behälter 1 verbundene Tropfkammer weist jeweils einen das Vorhandensein der Komponente detektierenden Sensor als zweiten Ultraschallsensor 9 auf. Diese sind die zweiten Bestandteile der Haltevorrichtung 7.

Die ersten Ultraschallsensoren 8 und die zweiten Ultraschallsensoren 9 sind Bestandteile von Klemmringen, die weiterhin Teile der Haltevorrichtung 7 sind. Weiterhin sind diese mit dem Datenverarbeitungssystem 4 verbunden.

Das Datenverarbeitungssystem 4 ist ein aus den Zeiten und den Signalen der ersten Ultraschallsensoren 8 den Fluss der Komponente ermittelndes Datenverarbeitungssystem 4. Das erfolgt mittels der Ermittlung der Differenzen und/oder der Mittelwerte aus den Zeiten und den Signalen der ersten Ultraschallsensoren 8. Daraus kann auch die Viskosität der in der Leitung fließenden Komponente und/oder der jeweilige in der Leitung fließende Stoff bestimmt werden.

Mittels der zweiten Ultraschallsensoren 9 wird das Vorhandensein der Komponente im Behälter 1 oder einer mit dem Behälter 1 verbundenen Tropfkammer detektiert. Damit wird das Nichtvorhandensein der Komponente über das Datenverarbeitungssystem 4 signalisiert und die zugeordnete Fördereinrichtung gestoppt. Der zweite Ultraschallsensor 9 besteht aus einem Ultraschallsender und einem Ultraschallempfänger für ein Durchschallungsverfahren.

Die Fig. 3 zeigt eine Spritzenpumpe 10 mit einem dritten Ultraschallsensor 11 in einer prinzipiellen Darstellung.

Die Fördereinrichtungen 2 sind angetriebene Spritzenpumpen 10, die jeweils über ein Ventil mit dem Sammelbehälter 3 für die Mischlösung verbunden sind. Der Rotor eines Schrittmotors als Antrieb der Spritzenpumpe 10 ist dazu über eine Gewindespindel und eine Bewegungsmutter auf der Gewindespindel mit dem Kolben der Spritze der Spritzenpumpe 10 gekoppelt, wobei die Gewindespindel ein Bestandteil eines Gestells ist. Eine derartige Realisierung ist bekannt.

Ein Bereich des zylindrischen Hohlraums der Spritze der Spritzenpumpe 10 für die Komponente kann einen das Vorhandensein der Komponente detektierenden dritten Ultraschallsensor 11 besitzen. Die Ultraschallsensoren 11 der Spritzenpumpen 10 sind mit dem Datenverarbeitungssystem 4 zusammengeschaltet.

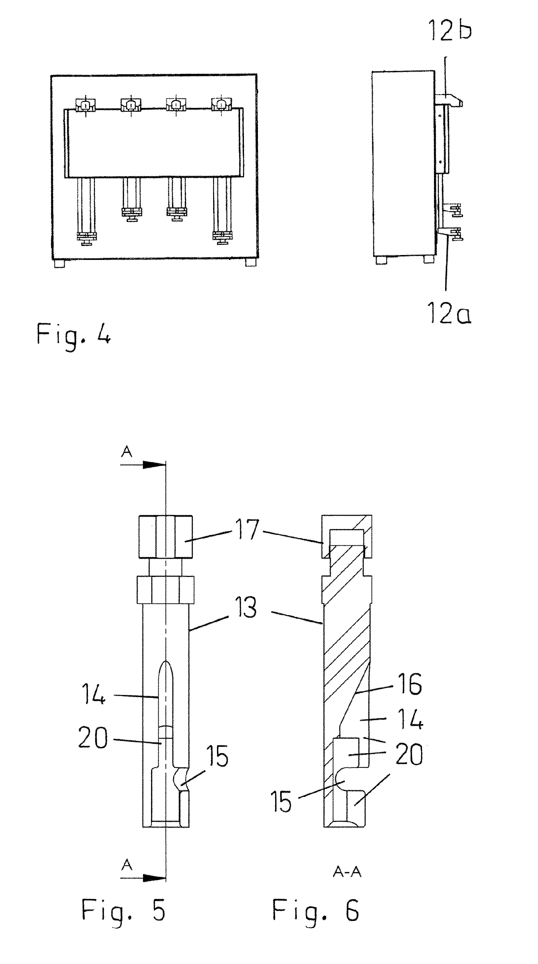

Die Fig. 4 zeigt eine Einrichtung für mehrere Spritzenpumpen 10 in einer prinzipiellen Darstellung.

Eine Einrichtung für die Spritzenpumpen 10 weisen die Antriebe und Halteeinrichtungen für die Spritzenpumpen 10 auf.

Das Datenverarbeitungssystem 4 ist mit wenigstens einer Dateneingabeeinrichtung, einem Bildschirm und mindestens einer Schnittstelle verbunden.

Eine Vorrichtung zur Bereitstellung einer aus mehreren Komponenten bestehenden Mischlösung kann in einer Ausführungsform im Wesentlichen aus
- wenigstens einem ersten Modul mit Behältern 1 mit flüssigen Komponenten und damit verbundenen Tropfkammern,
- mindestens einem zweiten Modul mit angetriebenen Spritzenpumpen 10 und mit elektrischen Verbindungselementen für Energie- und Datenleitungen für ein vor- und ein nachordenbares weiteres zweites Modul,
- einem dritten Modul als Waage für den die Mischlösung als Sammelbehälter 3 aufnehmenden Behälter und
- einem vierten Modul mit einem Datenverarbeitungssystem 4 als Steuereinrichtung bestehen.

Das erste Modul besitzt Aufnahmen oder Halteeinrichtungen für die Behälter 1. Der Behälter 1 oder der Behälter mit einer nachgeordneten Tropfkammer ist über ein Ventil oder ein Dreiwegeventil mit einer angetriebenen Spritzenpumpe 10 des zweiten Moduls gekoppelt. Die Spritzenpumpe ist eine Dosierpumpe, wobei der Kolben der Spritzenpumpe 10 an einen Antrieb gekoppelt ist. Damit wird die flüssige Komponente aus dem Behälter 1 oder der mit einem Behälter gekoppelten Tropfkammer angesaugt. Die Spritzenpumpe 10 ist weiterhin über ein weiteres Ventil oder das Dreiwegeventil bei entgegengesetzter Bewegung des Kolbens mit den die Mischlösung aufnehmenden Sammelbehälter 3 verbunden, so dass die Komponente in den Sammelbehälter 3 als den die Mischlösung aufnehmenden Behälter gedrückt wird. Die Ventile sind bekannte Rückschlagventile. Das Dreiwegeventil kann aus zwei miteinander gekoppelten Rückschlagventilen bestehen. Bei diesen Varianten ist eine Ansteuerung der Ventile nicht notwendig. Das zweite Modul weist in diesem Ausführungsbeispiel dazu vorzugsweise vier Spritzenpumpen auf. Eine Vorrichtung kann natürlich auch mehrere zweite Module aufweisen.

Die Spritze der Spritzenpumpe befindet sich in zwei Haltern 12. Diese besitzen eine U-Form, so dass die Spritze einfach ein- oder ausschiebbar ist. Dazu sind ein erster Halter 12a für die Kolbenstange in Verbindung mit dem Kolben und ein zweiter Halter 12b für entweder den Zylinder oder einen Adapter 13 in Verbindung mit dem Zylinder der Spritze der Spritzenpumpe 10 vorgesehen.

Der erste Halter 12a ist an einen Antrieb gekoppelt. Dazu ist der Rotor eines bekannten Schrittmotors als Antrieb über eine Gewindespindel und eine Bewegungsmutter auf der Gewindespindel mit dem ersten Halter 12a gekoppelt. Das zweite Modul besitzt weiterhin Einrichtungen zur Abstandsmessung zwischen wenigstens einem Fixpunkt und den Bewegungsmuttern. Weiterhin kann dieses zweite Modul als Basismodul ein Netzteil zur Energieversorgung der Antriebe, der Einrichtungen zur Abstandsmessung und der Steuereinrichtung als drittes Modul aufweisen. Das Netzteil kann aber auch ein separates Gerät sein.

Die Fig. 5 und Fig. 6 zeigen einen Adapter 13 für eine Spritze einer Spritzenpumpe 10 in einer prinzipiellen Darstellung und in einer prinzipiellen Schnittdarstellung.

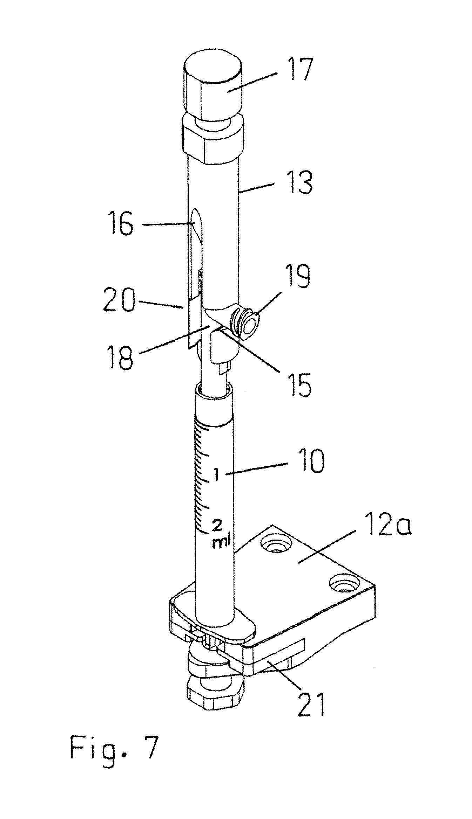

Die Fig. 7 zeigt einen Adapter 13 in Verbindung mit einer Spritze einer Spritzenpumpe 10 in einer prinzipiellen Darstellung.

Der Adapter 13 ist ein Zylinder in Verlängerung der Spritze in Längsrichtung. Der Zylinder weist eine Vertiefung 15 und eine Längsnut 20 für einen T-Verbinder 18 oder ein Dreiwegeventil und die Aufnahme 14 als Weiterführung der Längsnut 20 auf. Das Dreiwegeventil kann ebenfalls eine T-Form besitzen. Die Aufnahme 14 besitzt eine Schräge 16, so dass die Schlauchleitung nach außen geführt ist. Der Zylinder weist nach der Schräge 16 eine Befestigungseinrichtung als Schraubverbindung mit einer Befestigungsmutter 17 für den zweiten Halter 12b auf. Die Vertiefung 15 zur lösbaren Fixierung eines Leitungsstückes 19 des T-Verbinders 18 oder Dreiwegeventils und die Längsnut 20 sind ein Hohlraum, wobei die Vertiefung 15 und die Längsnut 20 winklig zueinander angeordnet sind.

In einer ersten Variante sind die vom Zylinder der Spritze nach außen geführte Schlauchleitung mit dem Behälter 1 oder der mit dem Behälter 1 verbundenen Tropfkammer und die mit dem Leitungsstück 19 in der Vertiefung 15 verbundene Schlauchleitung mit dem Sammelbehälter 3 verbunden. In einer zweiten Variante sind die vom Zylinder der Spritze nach außen geführte Schlauchleitung mit dem Sammelbehälter 3 und die mit dem Leitungsstück in der Vertiefung 15 verbundene Schlauchleitung mit einem Behälter 1 oder einer Tropfkammer in Verbindung mit einem Behälter 1 verbunden.

Der erste Halter 12a weist ein Reduzierstück 21 für die Öffnung des Halters 12a auf. Damit kann eine kleinere Spritze einfach mit den Haltern 12 für ansonsten große Spritzen verbunden werden.

Der T-Verbinder 18 besitzt drei Anschlüsse, wobei das Leitungsstück 19 in der Vertiefung 15 der erste Anschluss ist, der zweite Anschluss mit der Spritze verbunden ist und der dritte Anschluss in der Längsnut 20 angeordnet ist.

Das dritte Modul ist im Wesentlichen eine Waage für den Sammelbehälter 3.

Die Steuereinrichtung als viertes Modul ist ein bekanntes Datenverarbeitungssystem 4 mit wenigstens einer Dateneingabeeinrichtung und einem Bildschirm. Der Bildschirm als sogenannter Touchscreen kann auch die Dateneingabeeinrichtung beinhalten.

Die Steuereinrichtung kann mit Schaltern für die Energieversorgung der Antriebe der Spritzenpumpen, den Einrichtungen zur Abstandsmessung und der Waage zusammengeschaltet sein. Über die Schalter wird der jeweilige Stromkreis aus Antrieb und Netzteil geschlossen oder geöffnet. Im Zusammenhang mit den Einrichtungen zur Abstandsmessung sind die Positionen der Kolben der Spritzen der Spritzenpumpen und damit die durch die Spritzenpumpen zu fördernden Volumina der Komponenten bestimmbar.

Kabel als Daten- und/oder Energieleitungen in Verbindung mit Steckverbindern an den Modulen können den Betrieb der Vorrichtung gewährleisten.

### Bezugszeichen

- 1: Behälter
- 2: Fördereinrichtung
- 3: Sammelbehälter
- 4: Datenverarbeitungssystem
- 5: erste Leitung
- 6: zweite Leitung
- 7: Haltevorrichtung
- 8: Ultraschallsensor
- 9: detektierender Sensor
- 10: Spritzenpumpe
- 11: Ultraschallsensor
- 12: Halter
- 13: Adapter
- 14: Aufnahme
- 15: Vertiefung
- 16: Schräge
- 17: Befestigungsmutter
- 18: T-Verbinder
- 19: Leitungsstück
- 20: Längsnut
- 21: Reduzierstück

## Patentansprüche

1. Vorrichtung zur Bereitstellung einer aus mehreren Komponenten bestehenden Mischlösung mit
- Behältern (1) mit flüssigen Komponenten und damit über erste Leitungen (5) verbundenen Fördereinrichtungen (2) für die Komponenten,
- einem Sammelbehälter (3) für die Mischlösung über zweite Leitungen (6) in Verbindung mit den Fördereinrichtungen (2),
- jeweils mindestens vier Ultraschallsensoren (8) als Sender-Detektor-Kombinationen für eine Komponente einer Leitung kreuzweise durchdringende Schallwellen, wobei die Ultraschallsensoren (8) sowohl Sender als auch Empfänger sind,
- einem mit den Fördereinrichtungen (2) und den Ultraschallsensoren (8) verbundenen Datenverarbeitungssystem (4), wobei das Datenverarbeitungssystem (4) ein aus den Zeiten und den Signalen der Ultraschallsensoren (8) jeweils als Sender und Empfänger den jeweiligen Fluss der Komponente in der Leitung ermittelndes, mit vorbestimmten Mengen der Komponenten vergleichendes und im Ergebnis die jeweilige Fördereinrichtung (2) entsprechend der vorbestimmten Menge der Komponente an der Mischlösung steuerndes Datenverarbeitungssystem (4) ist.

2. Vorrichtung nach Patentanspruch 1, **dadurch gekennzeichnet, dass** das Datenverarbeitungssystem (4) ein aus den Zeiten und den Signalen der Ultraschallsensoren (8) die jeweilige Viskosität der in der Leitung fließenden Komponente ermittelndes Datenverarbeitungssystem (4) ist.

3. Vorrichtung nach Patentanspruch 1 und 2, **dadurch gekennzeichnet, dass** das Datenverarbeitungssystem (4) ein die Differenzen und/oder die Mittelwerte aus den Zeiten und den Signalen der Ultraschallsensoren (8) ermittelndes Datenverarbeitungssystem (4) ist, wobei daraus die jeweilige Viskosität der in der Leitung fließenden Komponente und/oder der jeweilige in der Leitung fließende Stoff bestimm- und zuordenbar ist.

4. Vorrichtung nach Patentanspruch 1, **dadurch gekennzeichnet, dass** der Behälter (1) und/oder eine mit dem Behälter (1) verbundene Tropfkammer jeweils einen das Vorhandensein der Komponente detektierender Sensor (9) aufweist und dass der das Vorhandensein der Komponente detektierende Sensor (9) mit dem Datenverarbeitungssystem (4) verbunden ist, wobei das Datenverarbeitungssystem (4) ein wenigstens das Nichtvorhandensein der Komponente signalisierendes und die zugeordnete Fördereinrichtung (2) stoppendes Datenverarbeitungssystem (4) ist.

5. Vorrichtung nach Patentanspruch 4, **dadurch gekennzeichnet, dass** der das Vorhandensein der Komponente detektierende Sensor (9) ein Ultraschallsensor ist, wobei die Leitung, die Tropfkammer und/oder der Behälter (1) zwischen dem Ultraschallsender und dem Ultraschallempfänger für ein Durchschallungsverfahren oder die Leitung, die Tropfkammer und/oder der Behälter (1) zwischen entweder den nebeneinander angeordneten Ultraschallsender und Ultraschallempfänger oder einer Kombination aus Ultraschallsender und Ultraschallempfänger und einem Reflektor für Ultraschallwellen für ein Reflexionsschallverfahren angeordnet sind.

6. Vorrichtung nach Patentanspruch 1 und 5, **dadurch gekennzeichnet, dass** sich die Ultraschallsensoren (8, 9) für eine Komponente jeweils in einem ring- oder u-förmigen Halteelement befinden und dass die Halteelemente getrennt voneinander fixierbar sind oder die Halteelemente zusammen ein Halter (7) sind.

7. Vorrichtung nach Patentanspruch 1, **dadurch gekennzeichnet, dass** die Fördereinrichtungen (2) angetriebene Spritzenpumpen (10) sind und dass die Spritzenpumpen (10) jeweils über ein Ventil mit dem Sammelbehälter (3) für die Mischlösung verbunden ist.

8. Vorrichtung nach Patentanspruch 7, **dadurch gekennzeichnet, dass** der Rotor eines Schrittmotors als Antrieb der Spritzenpumpe (10) über eine Gewindespindel und eine Bewegungsmutter auf der Gewindespindel mit dem Kolben (12) der Spritze der Spritzenpumpe (10) gekoppelt ist, wobei die Gewindespindel ein Bestandteil eines Gestells ist, dass eine Einrichtung zur Abstandsmessung zwischen wenigstens einem Teil des Gestells und der Bewegungsmutter zur Bestimmung der Position der Bewegungsmutter und der daraus resultierenden Position des Kolbens der Spritze der Spritzenpumpe (10) angeordnet ist und dass die Einrichtung zur Abstandsmessung mit dem Datenverarbeitungssystem (4) zusammengeschaltet ist, so dass das Datenverarbeitungssystem (4) ein das durch die Spritzenpumpe (10) geförderte Volumen der Komponente bestimmendes Datenverarbeitungssystem (4) ist.

9. Vorrichtung nach Patentanspruch 7, **dadurch gekennzeichnet, dass** jeweils auf wenigstens einem Bereich des zylindrischen Hohlraums der Spritze der Spritzenpumpe (10) für die Komponente ein das Vorhandensein der Komponente detektierender Ultraschallsensor (11) angeordnet ist und dass die Ultraschallsensoren (11) der Spritzenpumpen (10) mit dem Datenverarbeitungssystem (4) zusammengeschaltet sind.

10. Vorrichtung nach Patentanspruch 1, **dadurch gekennzeichnet, dass** das Datenverarbeitungssystem (4) mit wenigstens einer Dateneingabeeinrichtung, einem Bildschirm und mindestens einer Schnittstelle verbunden ist.

11. Vorrichtung nach Patentanspruch 1, **dadurch gekennzeichnet, dass** die Vorrichtung
- wenigstens ein erstes Modul mit Behältern (1) mit flüssigen Komponenten,
- mindestens ein zweites Modul mit angetriebenen Spritzenpumpen (10) und mit elektrischen Verbindungselementen für Energie- und Datenleitungen für ein vor- und ein nachordenbares weiteres zweites Modul, wobei die Spritzenpumpe (10) einen ersten mit dem Antrieb gekoppelten Halter (12a) in Verbindung mit dem Kolben und einen zweiten Halter (12b) für entweder den Zylinder der Spritzenpumpe (10) oder einen Adapter (13) in Verbindung mit dem Zylinder der Spritzenpumpe (10) aufweist und der Adapter (13) eine Vertiefung (15) zur lösbaren Fixierung für ein Leitungsstück (19) und eine Aufnahme (14) für einen Bereich einer Schlauchleitung besitzt,
- ein drittes Modul als Waage für den die Mischlösung aufnehmenden Behälter (7) und
- ein viertes Modul mit einer Steuereinrichtung als Datenverarbeitungssystem (4), wobei zum Einen jeweils ein Behälter (5) über wenigstens ein Ventil mit einer Spritzenpurrpe (10) und die jeweilige Spritzenpumpe (10) über das Ventil oder ein weiteres Ventil mit dem die Mischlösung aufnehmenden Behälter als Sammelbehälter (3) über Schlauchleitungen verbunden sind und zum Anderen die Antriebe der Spritzenpumpen (10) und die Waage mit der Steuereinrichtung zusammengeschaltet sind,
umfasst.

12. Vorrichtung nach Patentanspruch 11, **dadurch gekennzeichnet, dass** der Adapter (13) ein Zylinder in Verlängerung der Spritze der Spritzenpumpe (10) in Längsrichtung ist, dass der Zylinder eine Längsnut (20) für einen Verbinder oder ein Dreiwegeventil als Ventil und als Aufnahme (14) für den Bereich der Schlauchleitung aufweist, dass die Aufnahme (14) eine Schräge (16) besitzt, so dass die Schlauchleitung nach außen geführt ist, dass der Zylinder nach der Schräge (16) eine Befestigungseinrichtung für den zweiten Halter (12b) aufweist und dass die Vertiefung (15) zur lösbaren Fixierung des Leitungsstückes (19) des Verbinders oder Dreiwegeventils und die Längsnut (20) ein Hohlraum sind und die Vertiefung (15) und Längsnut (20) winklig zueinander angeordnet sind.

13. Vorrichtung nach Patentanspruch 12, **dadurch gekennzeichnet, dass** die vom Zylinder nach außen geführte Schlauchleitung mit einem Behälter (1) oder einer Tropfkammer in Verbindung mit einem Behälter (1) und die mit dem Leitungsstück in der Vertiefung (15) verbundene Schlauchleitung mit dem Sammelbehälter (3) oder dass die vom Zylinder nach außen geführte Schlauchleitung mit dem die Mischlösung aufnehmenden Behälter als Sammelbehälter (3) und die mit dem Leitungsstück in der Vertiefung (15) verbundene Schlauchleitung mit einem Behälter (1) oder einer Tropfkammer in Verbindung mit einem Behälter (1) verbunden sind.

14. Vorrichtung nach den Patentansprüchen 12 und 13, **dadurch gekennzeichnet, dass** der Verbinder ein T-Verbinder (18) mit drei Anschlüssen ist, dass das Leitungsstück in der Vertiefung (19) der erste Anschluss ist, dass der zweite Anschluss mit der Spritzenpumpe (10) verbunden ist und dass sich der dritte Anschluss in der Längsnut (20) befindet.

15. Vorrichtung nach den Patentansprüchen 11 bis 14, **dadurch gekennzeichnet, dass** der erste Halter (12a) ein Reduzierstück (21) für die Öffnung aufweist.
